(19) Europäisches Patentamt

European Patent Office

Office européen des brevets

(11) **EP 1 526 380 A1**

(12) **EUROPEAN PATENT APPLICATION**

(43) Date of publication:
**27.04.2005 Bulletin 2005/17**

(51) Int Cl.⁷: $G01N\ 33/574$

(21) Application number: **03380236.4**

(22) Date of filing: **21.10.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(71) Applicant: **Progenika Biopharma, S.A.
48160 Derio (Vizcaya) (ES)**

(72) Inventors:
 • **Del Amon Iribarren, Jokin
  48160, Derio Vizcaya (ES)**
 • **Junquera Sanchez-Vallejo, Corina
  48160, Derio Vizcaya (ES)**
 • **Ochoa Garay, Jorge
  48160, Derio Vizcaya (ES)**
 • **Escuredo Garcia de Galdeano, Pedro
  48160, Derio Vizcaya (ES)**
 • **Santa Cruz, Simon
  48160, Derio Vizcaya (ES)**
 • **Martinez Martinez, Antonio
  48160, Derio Vizcaya (ES)**

 • **Simon Buela, Laureano
  48160, Derio Vizcaya (ES)**
 • **Arguelles Sanchez, Maria Eladia
  Oviedo (ES)**
 • **De los Toyos Gonzalez, Juan Ramon
  Oviedo (ES)**
 • **Barneo Serra, Luis
  Oviedo (ES)**

(74) Representative: **Carpintero Lopez, Francisco
Herrero & Asociados, S.L.,
Alcalá, 35
28014 Madrid (ES)**

Remarks:
 The sequence listing, which is published as annex
 to the application documents, was filed after the date
 of filing. The applicant has declared that it does not
 include matter which goes beyond the content of the
 application as filed.

(54) **Methods for in vitro diagnosis and in vitro prognosis of cancer of the pancreas and for the development of drugs against cancer of the pancreas**

(57)  The present invention refers to an *in vitro* method to detect a cancer of the pancreas, especially a ductal adenocarcinoma of the pancreas, in an individual, to determine the stage or severity of this cancer in an individual or to monitor the effect of therapy administered to an individual with this cancer; to screen for, identify, develop and evaluate the efficacy of therapeutic compounds against this cancer in order to develop new medicinal products, and also agents that inhibit the expression and/or activity of the EFNB2 protein and/or the EDNRA protein, and/or the effects of this expression.

**EP 1 526 380 A1**

SEQUENCE LISTING

<110> Medplant genetics S.L.
<120> METHODS FOR IN VITRO DIAGNOSIS AND IN VITRO PROGNOSIS OF CANCER OF THE PANCREAS AND FOR THE DEVELOPMENT OF DRUGS AGAINST CANCER OF THEPANCREAS

<130> 2003870

<140> EP 03380236.4
<141> 2003-10-21

<160> 28

<170> PatentIn version 3.1

<210> 1
<211> 23
<212> DNA
<213> Artificial

<220>
<221> misc_feature
<223> Primer for the PCR amplification of the cDNA of EFBN2 gene

<400> 1
agcttgttta acggcagtgt cat                                          23


<210> 2
<211> 20
<212> DNA
<213> Artificial

<220>
<221> misc_feature
<223> Primer for the PCR amplification of the cDNA of EFBN2 gene

<400> 2
gcagcaattt ggcaaccttt                                              20


<210> 3
<211> 23
<212> DNA
<213> Artificial

<220>
<221> misc_feature
<223> Primer for the PCR amplification of the cDNA of EDNRA gene

<400> 3
tgcttgaatt gcaaggctaa gaa                                          23


<210> 4
<211> 23
<212> DNA
<213> Artificial

<220>
<221> misc_feature
<223> Primer for the PCR amplification of the cDNA of EDNRA gene

<400> 4
gcgccagtgg aataatagat ttg                                          23


<210> 5
<211> 25

```
<212>  DNA
<213>  Artificial

<220>
<221>  misc_feature
<223>  Probe corresponding to the set of probes 202668_at

<400>  5
ggaatttgca ccatcatgtt tcagt                                    25


<210>  6
<211>  25
<212>  DNA
<213>  Artificial

<220>
<221>  misc_feature
<223>  Probe corresponding to the set of probes 202668_at

<400>  6
tactgtctat ggatttgggg tgtta                                    25


<210>  7
<211>  25
<212>  DNA
<213>  Artificial

<220>
<221>  misc_feature
<223>  Probe corresponding to the set of probes 202668_at

<400>  7
gttacagtag ccttattcac ctttt                                    25


<210>  8
<211>  25
<212>  DNA
<213>  Artificial

<220>
<221>  misc_feature
<223>  Probe corresponding to the set of probes 202668_at

<400>  8
ggcttttctt acccagattg tgtac                                    25


<210>  9
<211>  25
<212>  DNA
<213>  Artificial

<220>
<221>  misc_feature
<223>  Probe corresponding to the set of probes 202668_at

<400>  9
ttagctacag cttgtttaac ggcag                                    25


<210>  10
<211>  25
<212>  DNA
<213>  Artificial

<220>
```

<221> misc_feature
<223> Probe corresponding to the set of probes 202668_at

<400> 10
tgtttaacgg cagtgtcatt cccct                          25

<210> 11
<211> 25
<212> DNA
<213> Artificial

<220>
<221> misc_feature
<223> Probe corresponding to the set of probes 202668_at

<400> 11
cattcccctt tgcactgtaa tgagg                          25

<210> 12
<211> 25
<212> DNA
<213> Artificial

<220>
<221> misc_feature
<223> Probe corresponding to the set of probes 202668_at

<400> 12
aattgctgca tatttgtgcc gtaat                          25

<210> 13
<211> 25
<212> DNA
<213> Artificial

<220>
<221> misc_feature
<223> Probe corresponding to the set of probes 202668_at

<400> 13
taaatctgct ttagtttcac attgc                          25

<210> 14
<211> 25
<212> DNA
<213> Artificial

<220>
<221> misc_feature
<223> Probe corresponding to the set of probes 202668_at

<400> 14
tagtttcaca ttgcagttag cccca                          25

<210> 15
<211> 25
<212> DNA
<213> Artificial

<220>
<221> misc_feature
<223> Probe corresponding to the set of probes 202668_at

<400> 15

```
aatccgtgaa gtcacattcc acatc                                    25


<210>   16
<211>   25
<212>   DNA
<213>   Artificial

<220>
<221>   misc_feature
<223>   Probe corresponding to the set of probes 204464_s_at

<400>   16
gtcatattgt ttcctgtgct ggagc                                    25


<210>   17
<211>   25
<212>   DNA
<213>   Artificial

<220>
<221>   misc_feature
<223>   Probe corresponding to the set of probes 204464_s_at

<400>   17
tgatatttct ttcagacttc gccag                                    25


<210>   18
<211>   25
<212>   DNA
<213>   Artificial

<220>
<221>   misc_feature
<223>   Probe corresponding to the set of probes 204464_s_at

<400>   18
gacttcgcca gacagattgc tgata                                    25


<210>   19
<211>   25
<212>   DNA
<213>   Artificial

<220>
<221>   misc_feature
<223>   Probe corresponding to the set of probes 202464_s_at

<400>   19
ataacatcag gttccagttg cttga                                    25


<210>   20
<211>   25
<212>   DNA
<213>   Artificial

<220>
<221>   misc_feature
<223>   Probe corresponding to the set of probes 204464_s_at

<400>   20
aagaagtact gcccttttgt gtgtt                                    25


<210>   21
```

```
<211>  25
<212>  DNA
<213>  Artificial

<220>
<221>  misc_feature
<223>  Probe corresponding to the set of probes 204464_s_at

<400>  21
ttattccact ggcgcatcat atgca                                          25


<210>  22
<211>  25
<212>  DNA
<213>  Artificial

<220>
<221>  misc_feature
<223>  Probe corresponding to the set of probes 204464_s_at

<400>  22
ggttcacacc attttgttta gacaa                                          25


<210>  23
<211>  25
<212>  DNA
<213>  Artificial

<220>
<221>  misc_feature
<223>  Probe correspopnding to the set of probes 204464_s_at

<400>  23
aggcgtcaac gtgcatttta tttat                                          25


<210>  24
<211>  25
<212>  DNA
<213>  Artificial

<220>
<221>  misc_feature
<223>  Probe correpsonding to the set of probes 204464_s_at

<400>  24
tttccaattt ctacctttac tacat                                          25


<210>  25
<211>  25
<212>  DNA
<213>  Artificial

<220>
<221>  misc_feature
<223>  Probe correspopnding to the set of probes 204464_s_at

<400>  25
ctacctttac tacatctttt caaca                                          25


<210>  26
<211>  25
<212>  DNA
<213>  Artificial
```

```
<220>
<221>  misc_feature
<223>  Probe corresponding to the set of probes 204464_s_at

<400>  26
aggccctgag ttggcagtgg cccat                                    25


<210>  27
<211>  17
<212>  DNA
<213>  Artificial

<220>
<221>  misc_feature
<223>  Primer for the PCR amplificaction of the cDNA of human ribosomal
       protein L10 gene

<400>  27
tgcgatggct gcacaca                                             17


<210>  28
<211>  23
<212>  DNA
<213>  Artificial

<220>
<221>  misc_feature
<223>  Primer for the PCR amplification of the cDNA of human ribosomal
       protein L10 gene

<400>  28
tcccttagag caacccatac aac                                      23
```

**Description**

**FIELD OF THE INVENTION**

The present invention refers to an *in vitro* method to detect the presence of a cancer of the pancreas in an individual, to determine the stage or severity of this cancer in the individual, or to monitor the effect of therapy administered to an individual with the said cancer; to screen for, identify, develop and evaluate the efficacy of therapeutic compounds for this cancer in an attempt to develop new medicinal products and to agents that inhibit expression of the Ephrin-B2 protein and/or the EDNRA protein, and/or the effects of this expression.

**BACKGROUND OF THE INVENTION**

Cancer of the pancreas was the cause of more than 220,000 deaths world-wide and more than 3,600 in Spain during the year 2,000 (GLOBOCAN). The clinical behaviour of pancreatic cancer is homogeneous and always unfavourable and there are no clear differences in survival in the different stages. Only very few patients with pancreatic cancer experience favourable outcomes. A possible explanation for this is that even in patients with small tumours, classified as stage I, the disease has spread beyond the pancreas. Its diagnosis in the initial stages, except for exceptional cases, is very difficult. Of patients diagnosed, 75% present the disease in advanced stages (Stages III and IV). It is a highly aggressive neoplasm resistant to cytostatic treatments and only between 1 and 4% of cases are alive five years after diagnosis corresponding to cases in which the tumour is localised and has been completely extirpated (Warshaw A.L., and Fernandes del Castillo C., N. Eng. J. Med., 1992, 326:455-465; Ahlgren J.D., Semin. Oncol. 1996, 23:241-250). For these reasons, the development of early diagnostic procedures and effective therapies are crucial to control this disease (Byungwoo R., et al., Cancer Res., 2002, 62:819-826).

Many of the genes and proteins involved in the development of the cancer of the pancreas are still unknown. Identification of genes and proteins expressed differentially in association with the invasive tumoral process could lead to the development of biological markers and therapeutic targets that could be very valuable in the diagnosis, prognosis and treatment of this disease.

Erythropoietin-producing hepatocellular markers (EPH) are a family of tyrosine-kinase receptors that are activated on interaction with their ligands, the ephrins. Both the ephrins and their ligands are involved in basic processes of nervous system development (Tessier-Lavigne M., 1995, Cell, 82:345-348; Winslow J.W., et al., 1995, Neuron, 14: 973-981; Lumsden A., and Krumlauf R., 1996, Science, 274:1109-1115; Magal E., et al., 1996; J. Neurosci. Res., 43: 735-744; Wang H.U., et al., 1997, Neuron, 18:383-396). Expression of ephrin and its receptors has also been associated with processes of angiogenesis during development of the circulatory system (Pandey A., et al., 1995, Science, 268: 567-569; Wang H.U., et al., 1998, Cell, 93:741-753; Adams R.H., et al., 1999, Genes Dev., 13:295-306; Wilkinson D. G., 2000, Int. Rev. Cytol., 196:177-244) and in human gastric cancer, cancer of the colon, lung and liver (Maru, Y. et al., Oncogene, 1990, 5:445-47; Kiyokawa E., et al., 1994, Cancer Res., 54:3645-3650; Easty D.J., et al., 1995, Cancer Res., 55:2528-2532; Tang X.X., et al., 1999, Clin. Cancer Res., 5:1491-1496).

Ephrin-B2 (EFNB2) is a transmembrane protein specifically expressed in arteries that reacts with the EPH B4 receptor, which is expressed more abundantly in veins, resulting in activation of the proliferation and migration stages of angiogenesis (Steinle J.J., et at., 2002, J. Biol.. Chem., 277:43830-43835; gerety S.S., and Anderson D.J., 2002, Development, 129:1397-1410). It has been previously shown that the gene ephrin-b2 (ephrin-b2; GeneBank code U16797. or efnb2), which encodes the protein ephrin-B2, is overexpressed in tumoral tissue samples from individuals with gastric cancer (Kataoka H. et al., 2002, J. Cancer res. Clin. Oncol., 128:343-348), neuroblastoma (Tang, X.X., et at., 2000, Proc. Natl. Acad. Sci., 97:19936-10941), primary and metastatic melanomas (Vogt T., et al., 1998, Clin. Cancer Res., 4:791-797) and small cell lung cancer (Tang, X.X., et al., 1999, Clin. Cancer Res., 5:455-460), but it has not been demonstrated previously that the EFNB2 gene, which encodes the protein EFNB2, or the protein EFNB2, is overexpressed in tumoral tissue from individuals with ductal adenocarcinoma of the pancreas.

Endothelins and their receptors are proteins with a strong vasoconstrictor activity on mature blood vessels. These proteins have also been shown to be involved in stimulating proliferation and migration of endothelial cells, myocytes and fibroblasts. These proteins have been associated with angiogenesis and their expression has been demonstrated in endothelial cells of tumours of ovary, colon, prostate, cervix, lung, uterus, Kaposi sarcoma, gliomas, meningiomas (Rosano L., et al., 2003, Cancer Res. 63:2447-53; Asham E., 2001, Br. J. Cancer., 85:1759-63; Peduto E. L., 2003, Br. J. Cancer, 88:788-95; Arducci M. A. J., 2003, Clin. Oncol. 21:679-89; Nelson J.B., 2003, J. Urol., 169: 1143-9; Gohji K.J., 2001, J. Urol. 165:1033-6; Ahmed S.I., 2000, Am. J. Respir. Cell Mol. Biol., 22:422-431; Breuiller-Fouche M., 1997, Obstet. Gynecol., 90:727-730; Bagnato A., 2002, Cancer Res. 62:6381; Bagnato A., 2001, Am. J. Pathol., 158:841-847; Harland S.P., 1998, Neurosurgery, 43:890-898; Tsutsumi K., 1994, J. Neurochem., 63: 2240-2247). However, it has not been shown previously that some of the genes that encode endothelins or their receptors, or some of these proteins, are overexpressed in tumoral tissue samples from individuals with pancreatic ductal adenocarcinomas.

Unexpectedly, the authors of the present invention have discovered, after thorough research and using different techniques (DNA-chips and quantitative RT-PCR to measure levels of gene expression and western-blotting to measure levels of protein expression), that expression of the efnb2 gene and the expression of the ednra gene, which encodes the endothelin receptor A (EDNRA), are elevated in the cancers of the pancreas when compared with non-tumoral expression in the same individuals or individuals with pancreatitis. The authors of the present invention have also discovered that the concentration of the EFNB2 protein is very high in pancreatic ductal adenocarcinomas, compared with non-tumoral expression in the same individuals, healthy individuals or individuals with pancreatitis. This makes EFNB2 and EDNRA useful targets for the development of new *in vitro* methods for diagnosis and prognosis and for the identification and development of therapeutic compounds for cancer of the pancreas, especially for pancreatic ductal adenocarcinomas.

The detection *in vitro* of high levels of expression of the efnb2 gene, the ednra gene, the EFNB2 protein, or the EDNRA protein, or any combination of these, in samples of pancreatic tissue or in serum samples of individuals permits early detection of pancreatic cancer.

The development of new drugs targeted specifically against the efnb2 gene, the ednra gene, the protein EFNB2, or the EDNRA protein, or any combination of these is a new approach to treat pancreatic ductal adenocarcinomas with such a poor prognosis.

The present invention, therefore, provides a highly sensitive *in vitro* method to detect the presence of cancer of the pancreas, especially a pancreatic ductal adenocarcinoma, to determine the stage or severity of this carcinoma in an individual or to monitor the effect of therapy administered to an individual with the said carcinoma, based on the detection and/or quantification of the EFNB2 protein, of the efnb2 mRNA or of the corresponding efnb2 cDNA, of the EDNRA protein, of the ednra mRNA, or of the corresponding ednra cDNA, or of any combination of these in a sample of an individual. Also, the present invention provides targets or tools for the screening, identification, development and evaluation of the efficacy of therapeutic compounds for the treatment of cancer of pancreas, especially for ductal adenocarcinoma of the pancreas. Finally, the invention provides agents characterised by the fact that they inhibit expression and/or activity of the EFNB2 protein and/or the EDNRA protein for the treatment of cancer of pancreas, especially for pancreatic ductal adenocarcinomas.

## SUMMARY OF THE INVENTION

The object of the present invention is to develop an *in vitro* method to detect the presence of cancer of the pancreas, especially of pancreatic ductal adenocarcinomas, to determine the stage or severity of this cancer in the individual or to monitor the effect of the therapy administered to an individual with this cancer.

A second object of the present invention is an *in vitro* method to screen for, identify, develop and evaluate the efficacy of compounds to treat cancer of the pancreas, especially pancreatic ductal adenocarcinomas.

An additional object of the invention lies in the use of sequences derived from the efnb2 gene to establish the diagnosis and *prognosis in vitro* of cancer of the pancreas, especially pancreatic ductal adenocarcinoma, and to screen for, identify, develop and evaluate the efficacy of compounds for the treatment of this cancer.

Another object of the present invention is to provide agents characterised because they inhibit expression and/or activity of the protein EFNB2, and/or of the EDNRA protein, to treat cancer of the pancreas, especially pancreatic ductal adenocarcinoma.

Finally, another object of this invention is a pharmaceutical composition consisting of one or several therapeutic agents together with an acceptable pharmaceutical excipient to treat cancer of the pancreas, especially pancreatic ductal adenocarcinoma.

## DESCRIPTION OF THE DRAWINGS

**Figure 1:** Denaturation curve of the PCR products amplified from the target efnb2 gene (Tm = 76°C) and of the reference gene, ribl10 (Tm=84°C), in experiments to measure gene expression by quantitative RT-PCR in real time in pancreas samples.

**Figure 2:** Calculation of the amplification efficiency of the PCR reactions of efnb2, in experiments to measure gene expression by quantitative RT-PCR in real time in pancreas samples.

**Figure 3:** Calculation of the amplification efficiency of the PCR reactions of ribl10, in experiments to measure gene expression by quantitative RT-PCR in real time in pancreas samples.

**Figure 4:** Denaturation curve of the PCR product of the target gene, ednra (Tm = 77°C) and the reference gene, ribl10 (Tm=84°C), in experiments to measure gene expression by quantitative RT-PCR in real time in pancreas samples.

**Figure 5:** Calculation of the amplification efficiency of PCR reactions of ednra, in experiments to measure gene expression by quantitative PCR in real time in pancreas samples.

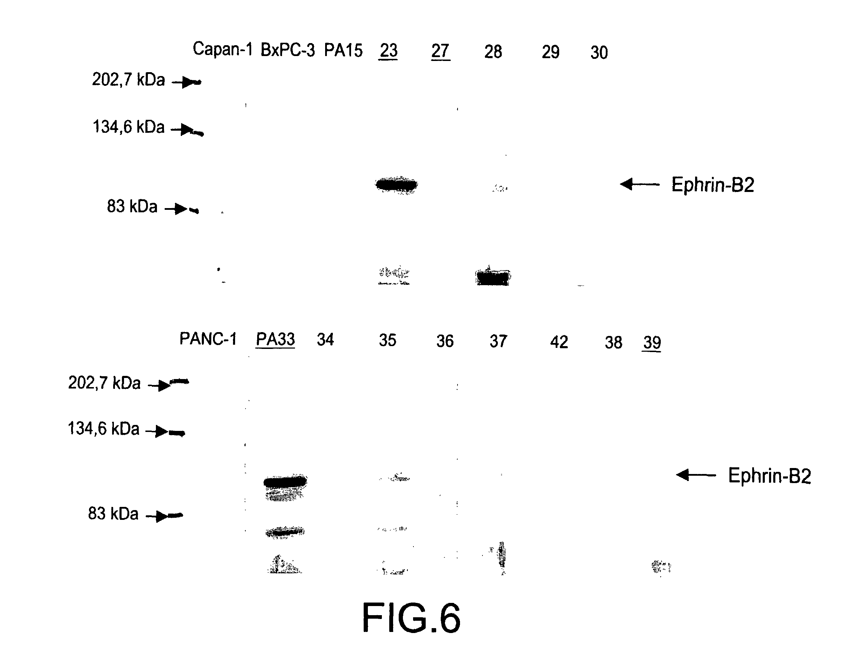

**Figure 6:** Results of the analysis of expression of EFNB2 protein in human pancreas samples by western transfer. Two healthy pancreas samples were analysed (samples number 15 and 42), four non-tumorous pancreas samples from patients with tumours (34, 35, 36 and 37), three apparently non-tumoral samples of inflamed pancreas from patients with tumour and pancreatitis (28, 29 and 30), two individual samples from patients with stage I pancreatic ductal adenocarcinoma (27 and 33) and one from a patient with stage IV pancreatic ductal adenocarcinoma (23). Finally, a healthy lung sample (38) and a pulmonary metastasis of pancreatic adenocarcinoma (39) were studied. A total of 40 μg of protein extract was used in each case. Membranes were developed with anti-EFNB2 antibody. Films were exposed for 1 min EFNB2 appeared as an immunoreactive band with an apparent molecular weight of 120 kDa.

## DETAILED DESCRIPTION OF THE INVENTION

To facilitate the comprehension of the present patent application we give the meanings of some terms and expressions in the context of the invention:

The terms "subject" or "individual" refer to animals of mammalian species and include, but are not restricted to, pets, primates and humans. The subject is preferably a male or female human of any age or race.

The terms "cancer" refers to the disease that is tipically characterised by an uncontrolled proliferation of abnormal cells capable of invading adjacent tissues and spreading to distant organs.

The term "cancer of the pancreas" or "cancer in the pancreas" or "adenocarcinoma of the pancreas" refers to any malign proliferative disorder of pancreatic ductal cells.

The term "tumour" refers to any abnormal mass of tissue generated by a neoplasic process, whether this is benign (non cancerous) or malignant (cancerous).

The term "gene" refers to a molecular chain of deoxyribonucleotides that encodes a protein.

The term "DNA" refers to deoxyribonucleic acid. A DNA sequence is a sequence of deoxyribonucleotides.

The term "cDNA" refers to a nucleotide sequence complementary to a sequence of mRNA.

The term "RNA" refers to ribonucleic acid. An RNA sequence is a sequence of ribonucleotides.

The term "mRNA" refers to messenger ribonucleic acid, which is the fraction of total RNA, which translates to proteins.

The term "mRNA transcript of" refers to the RNA product transcribed from the corresponding gene (DNA) into mRNA, as a first step in the expression and translation to protein.

The term "nucleotide sequence" or "nucleotidic sequence" refers either to a sequence of ribonucleotides (RNA) or a sequence of deoxyribonucleotides (DNA).

The term "protein" indicates at least one molecular chain of amino acids linked through either covalent or non-covalent bonds. The term includes all forms of post-translational modifications, for example glycosylation, phosphorilation or acetylation.

The terms "peptide" and "polypeptide" refer to molecular chains of amino acids that represent a protein fragment. The terms "protein" and "peptide" are used indistinguishably.

The term "antibody" refers to a glycoprotein that exhibits a specific binding activity for a specific protein called an "antigen". The term "antibody" refers to monoclonal or polyclonal antibodies, either intact or fragments derived from them; and includes human antibodies, humanised antibodies and antibodies of non-human origin.

The "monoclonal antibodies" are homogeneous, highly specific antibody populations directed against a single antigenic site or "determinant". "Polyclonal antibodies" include heterogeneous antibody populations that are directed against different antigenic determinants.

The term "epitope", as it is used in the present invention, refers to an antigenic determinant of a protein, which is the sequence of amino acids of the protein that a specific antibody recognises.

The term "solid phase", as it is used in the present invention refers to a nonaqueous matrix to which the antibody can bind. Examples of materials for the solid phase include glass, polysaccharides (for example agarose), polyacrylamide, polystyrene, polyvinylic alcohol and silicons. Examples of solid phase forms are the well of a plate or a purification column.

The term "oligonucleotide primer" and "primer" are used interchangeably in the present invention, and are used to refer to nucleotide sequences, that are complementary to target nucleotide sequences of the efnb2 or ribl 10 genes. Each primer hybridises with its target nucleotide sequence and acts as an initiation site for DNA polymerisation catalysed by DNA polymersase, RNA polymerase or reverse transcriptase.

The term "probe", as it is used in the present invention, refers to a nucleotide sequence complementary to a nucleotide sequence derived from the efnb2 gene that can be used to detect this nucleotide sequence derived from the efnb2 gene.

The term "therapeutic target" refers to nucleotide or peptide sequences against which a drug or therapeutic compound can be designed and clinically applied.

The term "antagonist" refers to any molecule that inhibits the biological activity of the antagonised molecule. Examples of antagonistic molecules include, among others, proteins, peptides, variations of natural peptide sequences and small organic molecules (with a molecular weight lower than 500 Daltons).

The present invention is based on the discovery that both gene expression of efnb2 and ednra, and the concentration of the EFNB2 and EDNRA proteins are increased in ductal adenocarcinoma of the pancreas of an individual.

Therefore, the present invention first of all provides an *in vitro* method to detect the presence of a cancer of the pancreas, especially of a ductal adenocarcinoma of the pancreas in an individual, to determine the stage or severity of this cancer in the individual, or to monitor the effect of the therapy administered to an individual with this cancer, that comprises:

a) detection and/or quantification either of EFNB2 protein, of the efnb2 mRNA or the corresponding cDNA, or of the EDNRA protein, of the of ednra mRNA or of the corresponding cDNA or of any combination of these in a sample of an individual and;

b) comparison of either the amount of EFNB2 protein, of the mRNA of the efnb2 gene or of the corresponding cDNA, or of the amount of EDNRA protein, of mRNA of the ednra gene, or of the corresponding cDNA or of any combination of these detected in a sample of an individual, with the respective amount either of the EFNB2 protein, of the mRNA of the efnb2 gene or of the corresponding cDNA, or with the amount of the EDNRA protein, of the mRNA of the ednra gene or of the corresponding cDNA or with any combination of these detected in the samples of control individuals or in previous samples of the same individual or with normal reference values.

The method provided by the present invention is highly sensitive and specific and is based on the fact that subjects or individuals diagnosed with cancer of the pancreas, especially with ductal adenocarcinoma of the pancreas, present high levels of mRNA transcribed from the efnb2 gene (*elevated levels of expression of the efnb2 gene*) or elevated levels of the protein coded by the efnb2 gene (*Protein EFNB2*), or elevated levels of mRNA transcribed from the ednra gene (*elevated levels of expression of the ednra gene*), or elevated levels of the protein coded by the ednra gene (*EDNRA* protein), in comparison with the corresponding levels in samples from subjects without a clinical history of this cancer.

The present method comprises a step in which a sample is obtained from the individual. Different liquid samples can be used such as: urine, blood, plasma, serum, pleural fluid, ascitic fluid, synovial fluid, bile, semen, gastric exudate or cerebrospinal fluid. The sample can also consist of tissue of the affected organ, such as the pancreas that can be obtained by any conventional method, preferably surgical resection.

Samples can be obtained from subjects previously diagnosed or not diagnosed with a specific type of cancer; or also from a subject receiving treatment, or who has previously received treatment for a cancer, especially for ductal adenocarcinoma of the pancreas.

The present method also comprises a step for extraction of the sample, either to obtain the extract of proteins from it or to obtain the extract of total RNA. One of these two extracts provides the working material for the next phase. The extraction protocols for total protein or total RNA are well known by experts in the area (Chomczynski P. et al., Anal. Biochem., 1987, 162: 156; Chomczynski P., Biotechniques, 1993, 15: 532).

Any conventional assay can be used in the context of the invention to detect a cancer, provided that it measures *in vitro* the levels of mRNA transcribed from the efnb2 gene or its complementary cDNA, the concentration of the Protein EFNB2, or the levels of mRNA transcribed from the ednra gene or its complementary cDNA, or the concentration of EDNRA protein, in samples collected from individuals to be studied and control individuals.

Therefore, this invention provides a method to detect the presence of a cancer of the pancreas, especially the presence of ductal adenocarcinoma of the pancreas in an individual, to determine the stage or severity of this cancer in an individual, or to monitor the effect of the therapy administered to an individual who presents this cancer, based either on measuring the levels of the EFNB2 protein or on measuring the level of expression of the EDNRA gene or the level of expression of the ednra gene.

If the aim is to detect the EFNB2 protein or the EDNRA protein, the method of the invention comprises a first step in which the protein extract of the sample is placed in contact with a composition of one or more specific antibodies against one or more epitopes of the EFNB2 protein or the EDNRA protein, and a second stage to quantify the complexes formed by antibodies and the EFNB2 protein or the EDNRA protein.

There is a wide range of immunological assays available to detect and quantify formation of specific antigen-antibody complexes; numerous competitive or non-competitive protein-binding assays have been described previously and a large number of these are available commercially. Hence, the EFNB2 protein or the EDNRA protein can be quantified with antibodies such as, for example: monoclonal antibodies, polyclonal antibodies, either intact or recombinant fragments of these, combibodies and Fab fragments or scFv of antibodies, specific against the protein EFNB2; these antibodies are human, humanised or of animal origin. The antibodies used in these assays can be labelled or unlabelled; the unlabelled antibodies can be used in agglutination assays; the labelled antibodies can be used in a wide range of assays. Marker molecules that can be used to label antibodies include radionucleotides, enzymes, fluofluorides, chemoluminescent reagents, enzymatic substrates or cofactors, enzymatic inhibitors, particles, colorants and derivatives. There are a wide variety of well known assays that can be used in the present invention, which use unlabelled antibodies (primary antibody) and labelled antibodies (secondary antibodies); these techniques include the western-blot or western transfer, ELISA (Enzyme-Linked immunosorbent assay), RIA (Radioimmunoassay), Competitive EIA (Competitive enzyme immunoassay), DAS-ELISA (Double antibody sandwich-ELISA), immunocytochemical and immunohistochemical techniques, techniques based on the use of biochips or protein microarrays that include specific antibodies or colloidal precipitation in formats such as *dipsticks.* Other ways to detect and quantify the EFNB2 protein or the EDNRA protein include affinity chromatography techniques, ligand binding assays or lectin binding assays.

The preferred immunoassay for the invention is a double antibody sandwich ELISA (*DAS-ELISA*). In this immunoassay any antibody or combination of antibodies can be used, specific against one or more epitopes of the EFNB2 protein or the EDNRA protein. As an example of one of the many possible formats of this assay, a monoclonal or polyclonal antibody, or a fragment of this antibody, or a combination of these antibodies, that cover a solid phase, is placed in contact with the sample to be analysed and is incubated for a specific time and in appropriate conditions to form the antigen-antibody complexes. After washing in appropriate conditions to eliminate non-specific complexes, an indicator reagent, consisting in a monoclonal or polyclonal antibody, or a fragment of this antibody, or a combination of these, bound to a signal generator compound, is incubated with the antigen-antibody complexes in appropriate conditions of time and temperature. Presence of the EFNB2 protein or the EDNRA protein in the sample to be analysed is detected and, if present, quantified and the signal generated is measured. The amount of EFNB2 protein or EDNRA protein present in the sample to be analysed is proportional to this signal.

When the aim is to detect mRNA or the cDNA corresponding to the efnb2 gene or the mRNA corresponding to the ednra gene and not the proteins that code this, the method of the invention to detect the carcinoma *in vitro* has several different steps. Hence, after obtaining the sample and extracting the total RNA, the method of the invention effects the detection of the mRNA or of the corresponding cDNA of the efnb2 gene or of the mRNA or the corresponding cDNA of the ednra gene, that comprises a first step of amplification of the RNA that is present in the extract of total RNA, or of the corresponding cDNA synthesised by reverse transcription of the mRNA; and a second step of quantification of the amplification product from either the mRNA or the cDNA of the efnb2 gene, or the mRNA or the cDNA of the ednra gene.

One example of mRNA amplification consists in retrotranscribing the mRNA into cDNA (RT), followed by Polymerase Chain Reaction (PCR), using oligonucleotide primers, using the primer sequences 5'-AGCTTGTTTAACGGCAGTGTCAT-3' and 5'- GCAGCAATTTGGCAACCTTT - 3' for efnb2 and 5'- TGCTTGAATTGCAAGGCTAAGAA -3' and 5'-GCGCCAGTGGAATAATAGATTTG -3' for ednra. PCR is a technique for the amplification of a specific nucleotide sequence (target) contained in a mixture of nucleotide sequences. In PCR, an excess of a pair of oligonucleotide primers is used that hybridise with complementary strands of the target nucleotide sequence. After this, an enzyme with polymerase activity (DNA Polymerase) extends each primer, using the target nucleotide sequence as a template. The extension products are, therefore, converted into target sequences, after dissociation of the original strand. New primer molecules hybridise and the polymerase extends them. The cycle is repeated to exponentially increase the number of target sequences. This technique is described in the patents US 4683195 and US 4683202. Many methods have been described previously to detect and quantify amplification products by PCR and any of these can be used in the invention. In a preferred method of the invention, the amplified product is detected by agarose gel electrophoresis as follows: five microliters of amplification product are separated by agarose gel electrophoresis at a concentration of 2%, in a TBE buffer 0.5x at 100 vdc for one hour. After electrophoresis the gel is stained with ethidium bromide and the amplification product is observed when the gel is illuminated with ultraviolet (uv) light. As an alternative to staining, a preferred method is to transfer the amplified product to a nylon membrane by *Southern blotting or Southern transfer* techniques to be detected with a specific cDNA probe of the efnb and/or ednra gene, appropriately labelled.

In another example, mRNA detection is performed following electrophoretic separation of mRNA by transferring the mRNA to a nylon membrane using transfer techniques such as northern-blot or northern transfer and detecting it with specific RNA probes or of the corresponding cDNA of the efnb gene or the mRNA or the corresponding cDNA of the ednra gene.

In one specific application, amplification and quantification of the mRNA corresponding to the efnb2 gene and/or amplification and quantification of the mRNA corresponding to the ednra gene, is carried out by quantitative RT-PCR in real time (Q-PCR).

The final step of the method of the invention to detect *in vitro* the presence of the cancer in a sample from an individual comprises comparing the amount of protein EFNB2, the amount of mRNA of the efnb2 gene or the amount of the corresponding cDNA or the amount of EDNRA protein, the amount of mRNA of the ednra gene, or the amount of corresponding cDNA detected in a sample of an individual, with the amount of protein EFNB2, the amount of mRNA of the efnb2 gene, the amount of corresponding cDNA, with the amount of EDNRA protein, the amount of mRNA of the ednra gene, the amount of corresponding cDNA detected in the samples of control subjects or in previous non-tumorous samples of the same individual or with normal reference values.

For its second object, the invention also provides a method *in vitro* to identify and evaluate the efficacy of therapeutic agents against a cancer of the pancreas, especially against a ductal adenocarcinoma of the pancreas that comprises:

a) placing into contact a culture of immortalised endothelial cells, with the candidate compound, in the appropriate conditions and the time required for these to interact,
b) detection and quantification of the expression levels of the efnb2 gene, the protein EFNB2, the ednra gene, the EDNRA protein, or any combination of these and
c) comparing these expression levels with those of the control culture of immortalised endothelial cells not treated with the candidate compound.

Quantification of the expression levels of the efnb2 gene, the protein EFNB2, the ednra gene or the EDNRA protein is done in a similar way to that described in the method of the invention to detect *in vitro* the presence of a cancer of the pancreas, especially of a ductal adenocarcinoma of the pancreas in an individual.
When an agent reduces the expression levels of the efnb2 gene and/or the ednra gene or reverses the effects of high expression of this gene, preferably reducing the levels of cellular proliferation, this agent becomes a candidate for the cancer therapy.
Therefore, another object of this invention refers to the use of nucleotide or peptide sequences derived from the efnb2 and/or ednra gene, in methods to screen for, identify, develop and evaluate the efficacy of therapeutic compounds against cancer of the pancreas, especially ductal adenocarcinoma of the pancreas. It is noteworthy, the recent importance given to screening methods based on the competitive or non-competitive binding of the potential therapeutic molecule to the therapeutic target.

Another object of this invention refers to the use of nucleotide or peptide sequences derived from the efnb2 and/or the ednra gene to detect the presence of a cancer of the pancreas, especially of a ductal adenocarcinoma of the pancreas, to determine the stage or severity of these cancers in the individual or to monitor the effect of the therapy administered to an individual with these cancers.
Another object of this invention consists in providing agents characterised because they inhibit expression and/or activity of the EFNB2 protein and/or the EDNRA protein. These agents, which can be identified and evaluated according to the present invention, can be selected from the group comprised by:

a) an antibody, or combination of antibodies, specific against one or more epitopes present in the EFNB2 protein or in the EDNRA protein, preferably a human or humanised monoclonal antibody. These can also be a fragment of antibody, a single chain antibody or an anti-idiotype antibody,
b) cytotoxic agents, such as toxins, molecules with radioactive atoms or chemotherapeutic agents, including, but not limited to, small organic and inorganic molecules, peptides, phospopeptides, antisense molecules, ribozymes, siRNAs, triple helix molecules etc. that inhibit expression and/or activity of the EFNB2 protein and/or the EDNRA protein, and
c) antagonistic compounds of the EFNB2 protein and/or the EDNRA protein that inhibit one or more of the functions of the EFNB2 protein and/or the EDNRA protein .

Finally, another object of the present invention is a pharmaceutical composition that includes a therapeutically effective amount of one or several of the previously mentioned agents together with one or more excipients and/or transporter substances. Also, this composition can contain any other active ingredient that inhibits the function of the EFNB2 and/or the EDNRA protein.
The excipients, transporter compounds and auxiliary substances must be pharmaceutically and pharmacologically tolerable so that they can be combined with other components of the formulation or preparation and not have any adverse effects on the organism treated. The pharmaceutical compositions or formulations include those that are suitable for oral or parenteral administration (including subcutaneous, intradermic, intramuscular or intravenous), although the best route of administration depends on the patient's condition. Formulations can also be in the form of single doses. Formulations are prepared according to well known pharmacological methods. Amounts of active substances to be administered vary depending on the characteristics of the therapy.

The following examples serve to illustrate the invention.

Example 1.- Differential analysis of the expression of the efnb2 gene and **the ednra gene in samples of pancreas tissue, using *Human Genome U133 DNA microarrays***

### 1.1. Material and methods

Microarrays. GeneChip Test 3 (Affymetrix, Santa Clara) microarrays were used, that permitted the quality of RNA to be tested before analysing expression with the GeneChip Human Genome U133A array (Affymetrix, Santa Clara), which represents 13,220 recorded complete gene sequences; the efnb2 gene is represented in the microarray by the set of probes 202668_at of Affymetrix, which are sense oligonucleotides 25 nucleotides long, designed on the basis of the Hs.30942 sequence of Unigene, or U16797 of GeneBank (Table 1); the ednra gene is represented in the microarray by the set of probes 204464_s_at of Affymetrix, which are sense nucleotides 25 nucleotides long, designed on the basis of the Hs.76252 sequence of Unigene or L06622 of GeneBank (Table 2).

## Table 1. Description of the probes corresponding to the set of probes 202668_at

| Consecutive order of probes | Zone of the interrogated reference sequence | Probe sequence (5'-3') |
|---|---|---|
| 1 | 3877 | GGAATTTGCACCATCATGTTTCAGT |
| 2 | 3929 | TACTGTCTATGGATTTGGGGTGTTA |
| 3 | 3950 | GTTACAGTAGCCTTATTCACCTTTT |
| 4 | 4008 | GGCTTTTCTTACCCAGATTGTGTAC |
| 5 | 4104 | TTAGCTACAGCTTGTTTAACGGCAG |
| 6 | 4116 | TGTTTAACGGCAGTGTCATTCCCCT |
| 7 | 4132 | CATTCCCCTTTGCACTGTAATGAGG |
| 8 | 4181 | AATTGCTGCATATTTGTGCCGTAAT |
| 9 | 4327 | TAAATCTGCTTTAGTTTCACATTGC |
| 10 | 4338 | TAGTTTCACATTGCAGTTAGCCCCA |
| 11 | 4371 | AATCCGTGAAGTCACATTCCACATC |

## Table 2. Description of the probes corresponding to the set of probes 204464_s_at

| Consecutive order of probes | Zone of the interrogated reference sequence | Probe sequence (5'-3') |
|---|---|---|
| 1 | 3517 | GTCATATTGTTTCCTGTGCTGGAGC |
| 2 | 3617 | TGATATTTCTTTCAGACTTCGCCAG |
| 3 | 3631 | GACTTCGCCAGACAGATTGCTGATA |
| 4 | 3704 | ATAACATCAGGTTCCAGTTGCTTGA |
| 5 | 3740 | AAGAAGTACTGCCCTTTTGTGTGTT |
| 6 | 3779 | TTATTCCACTGGCGCATCATATGCA |
| 7 | 3832 | GGTTCACACCATTTTGTTTAGACAA |
| 8 | 3937 | AGGCGTCAACGTGCATTTTATTTAT |
| 9 | 3998 | TTTCCAATTTCTACCTTTACTACAT |
| 10 | 4008 | CTACCTTTACTACATCTTTTCAACA |
| 11 | 4063 | AGGCCCTGAGTTGGCAGTGGCCCAT |

**Samples.** The samples studied were from clinically classified biopsies, obtained by surgical resection: biopsies of non-tumorous pancreatic tissue from individuals with ductal adenocarcinoma of the pancreas ("non-tumorous") (n = 1); biopsies of non-tumorous pancreatic tissue, but with pancreatitis, from individuals without ductal adenocarcinoma of the pancreas, but with chronic pancreatitis ("chronic pancreatitis") (n = 1); biopsies of non-tumorous pancreatic tissue, but with pancreatitis, from patients with ductal adenocarcinoma of the pancreas and pancreatitis ("pancreatitis") (n = 1); biopsies of tumorous pancreatic tissue, from patients with ductal adenocarcinoma of the pancreas ("tumour") (n = 10) in one of the following stages: Stage I, tumour limited to pancreas. Stage III, tumour spread to regional lymph nodes and Stage IVB, metastases in distant organs and tissues. All samples were clinically and histologically classified (grade and stage) in the Hospital Central de Asturias, the same hospital where they had been collected according to the precepts of the Helsinki declaration. Samples were frozen in liquid nitrogen immediately after extraction and stored at -80°C until analysis.

For each stage of tumour the following samples were analysed:

- Stage I tumour:    7 samples
- Stage III tumour:    1 sample
- Stage IV tumour:    2 samples

**GeneChip gene expression analysis**

Analysis was done with total RNA from individual subjects. The 13 samples analysed are described in Table 3.

Table 3.

| Description of the samples analysed | | | |
|---|---|---|---|
| Sample Code | Type | TNM Classification | Stage |
| PA36 | Non-tumorous | - | - |
| PA29 | Pancreatitis | - | - |
| PA46 | Chronic Pancreatitis | - | - |
| PA02 | Tumour | T2N0M0 | I |
| PA03 | Tumour | T2N0M0 | I |
| PA17 | Tumour | T1bN0M0 | I |
| PA19 | Tumour | T1bN0M0 | I |
| PA25 | Tumour | T1N0M0 | I |
| PA27 | Tumour | T1N0M0 | I |
| PA33 | Tumour | T1bN0M0 | I |
| PA24 | Tumour | T3N1M0 | III |
| PA16 | Tumour | T2N1M1 | IV |
| PA23 | Tumour | T1bN1M1 | IV |

*CRNA synthesis*

Total RNA of each biopsy was obtained by homogenising the tissue in TRIzol® Reagent (Life Technologies), following the supplier's recommendations. The resulting total RNA was cleaned with the Rneasy kit (QIAGEN) (Chomczynski P. et al., Anal. Biochem., 1987, 162: 156; Chomczynski P., Biotechniques, 1993, 15: 532). Of each preparation of total RNA, 10 μg were used as starting material for synthesis of the first cDNA strand with the reverse transcriptase enzyme SuperScript™ II RNase (Life Technologies), using as a primer an oligonucleotide oligo-dT containing the sequence of the RNA polymerase promoter of phage T7. The second cDNA strand was synthesised using the enzymes DNA polymerase I of *E. coli* (Invitrogen Life Technologies), DNA ligase of *E. coli* (Invitrogen Life Technologies), Rnase H of *E. coli* (Invitrogen Life Technologies), and DNA polymerase of phage T4 (Invitrogen Life Technologies). The biotin labelled cRNA was synthesised using the ENZO BioArray™ HighYield™ Transcript Labeling Kit (Enzo Diagnostics Inc). After in *vitro* transcription, the nucleotides not incorporated were eliminated using the RNeasy columns (QIAGEN).

*Array Hybridization and scanning*

A total of 15 µg of each biotinylated cRNA were fragmented at 94°C for 35 minutes in a buffer solution containing 40 mM Tris-Acetate (pH 8.1), 100mM KOAc and 30mM MgOAc. The fragmented cRNA was mixed with hybridization buffer (100mM MES, 1M NaCl, 20 mM EDTA, 0.01% Tween 20) and heated to 99° for 5 minutes and then to 45° for 5 minutes, after which it was loaded in the Affymetrix array. The first array in which the hybridization was carried out was Test 3 of Affymetrix. With this array the quality of RNA can be tested before analysing expression in the Affymetrix® GeneChip® *Human Genome* 133 A (HG-U133A).

For hybridization, arrays were incubated in a rotary incubator at 45° for 16 hours with a constant rotation of 60 rpm. Washing and staining of each array was done in the Affymetrix® fluid station. A washing and staining programme was used that included:

- 10 x 2 washing cycles with SSPE-T 6x (0.9 m NaCl, 60 mM NaH$_2$PO4, 6 mM EDTA, 0.01 % Tween 20) at 25°C,
- 4x15 cycles with 0.1 mM MES, 0.1M NaCl, 0.01 % Tween 20 at 50°C,
- Staining with biotinylated cRNA with a phycoerythrin streptavidin conjugate (10 µg/ml Molecular Probes)
- 10 x 4 washing cycles with SSPE-T at 25C°
- Staining an anti-streptavidin conjugate for 10 minutes
- Staining a phycoerythrin-streptavidin conjugate (1 mg/ml, Molecular Probes) for 10 minutes
- 15 x 4 washing cycles with SSPE-T at 30C°

Arrays were scanned at 560 nm using a confocal microscope that uses laser emission (Agilent GeneArray Scanner). Analysis of intensity readings was done with the Microarray Suite 5.0 software. For comparison of arrays these were scaled to a total intensity of 100.

**1.2. Results**

Differential analysis of the expression of the efnb2 and ednra genes in biopsies of tumorous pancreatic tissue, compared to biopsies of non-tumorous pancreatic tissue, was done using the array comparison data obtained using the Affymetrix software. The parameters studied (in the order in which these appear in the list) were: i) Detection. This indicates whether the transcript is Present (P), Absent (A) or Marginal (M), ii) Change: This indicates whether expression of a specific transcript Increases (I), Decreases (D), Does not change (NC), Increases marginally (IM), or decreases marginally (DM), iii) Signal Log Ratio (SLR): This indicates the level of change in expression between the baseline (control) and the sample under test. This change is expressed as the log$_2$ of the ratio (*fold change* or number of times that this gene expression is elevated or repressed in the problem-tumoral sample versus a healthy control sample). An SLR value of 1 is considered to be significant (equivalent to a *fold change* of 2), for transcripts in which expression increases compared to control and of -1, for transcripts for which expression decreases compared to control.

**Table 4. Results obtained for the efnb2 gene. N. Acc. U16797. Result of comparisons with PA36 non-tumorous control sample**

| Sample | Detection | SLR | Change |
|---|---|---|---|
| Non-tumorous controls | | | |
| PA36 | A | - | - |
| PA29 | P | 2.1 | NC |
| PA46 | P | 1.7 | I |
| Stage I tumours | | | |
| PA02 | P | 2 | I |
| PA03 | P | 2.4 | I |
| PA17 | A | 0.2 | NC |
| PA19 | P | 2.8 | I |
| PA25 | P | 1.3 | NC |
| PA27 | P | 2.3 | I |
| PA33 | P | 2.2 | I |
| Stage III tumours | | | |
| PA24 | P | 2.6 | I |
| Stage IV tumours | | | |
| PA23 | P | 3.1 | I |
| PA16 | P | 4.7 | I |

**Table 5. Results obtained for the efnb2 gene. N. Acc. U16797. Results of comparisons with PA46 chronic pancreatitis control sample.**

| Sample | Detection | SLR | Change |
|---|---|---|---|
| Non-tumorous controls | | | |
| PA46 | P | - | - |
| PA29 | P | 0.6 | NC |
| PA36 | A | -1.7 | D |
| Stage I tumours | | | |
| PA02 | P | 1.3 | I |
| PA03 | P | 1.4 | I |
| PA17 | A | -0.1 | NC |
| PA19 | P | 1.3 | I |
| PA25 | P | 0 | NC |
| PA27 | P | 0.4 | NC |
| PA33 | P | 0.9 | I |
| Stage III tumours | | | |
| PA24 | P | 1.4 | I |
| Stage IV tumours | | | |
| PA23 | P | 1.5 | I |
| PA16 | P | 2.9 | I |

## Table 6. Results obtained for the ednra gene. N. Acc. L06622. Result of comparisons with PA36 non-tumorous control sample

| Sample | Detection | SLR | Change |
|---|---|---|---|
| **Non-tumorous controls** | | | |
| PA36 | P | - | - |
| PA29 | P | 1 | NC |
| PA46 | P | -0.9 | NC |
| **Stage I tumours** | | | |
| PA02 | P | 0.9 | NC |
| PA03 | P | 2.2 | I |
| PA17 | P | 1.5 | NC |
| PA19 | P | 1.8 | I |
| PA25 | P | 0.8 | NC |
| PA27 | P | 3.6 | I |
| PA33 | P | 1.1 | NC |
| **Stage III tumours** | | | |
| PA24 | P | 1.2 | NC |
| **Stage IV tumours** | | | |
| PA23 | P | 0.8 | NC |
| PA16 | P | 1.2 | NC |

### Table 7. Results obtained for the ednra gene. N. Acc L06622. Results of comparisons with PA46 chronic pancreatitis control sample.

| Sample | Detection | SLR | Change |
|--------|-----------|-----|--------|
| Healthy controls | | | |
| PA46 | P | - | - |
| PA29 | P | 2.1 | ↑ |
| PA36 | P | 0.9 | NC |
| Stage I tumours | | | |
| PA02 | P | 1.9 | MI |
| PA03 | P | 3.7 | ↑ |
| PA17 | P | 2.2 | ↑ |
| PA19 | P | 2.7 | ↑ |
| PA25 | P | 1.9 | ↑ |
| PA27 | P | 4 | ↑ |
| PA33 | P | 1.7 | ↑ |
| Stage III tumours | | | |
| PA24 | P | 2.1 | ↑ |
| Stage IV tumours | | | |
| PA23 | P | 1.7 | ↑ |
| PA16 | P | 2 | ↑ |

Differential analysis of the expression of the efnb2 gene in tumorous stages compared with the non-tumorous control (PA36), showed that expression levels of the efnb2 gene were four fold or higher (SLR≥2) in 5 of the 7 biopsies of Stage I pancreas tumours, in the biopsy of the Stage III tumour and in the 2 biopsies of the Stage IV tumours (Table 4). The differential expression analysis of the efnb2 gene compared to the control with chronic pancreatitis (PA46), showed that expression levels of the efnb2 gene were increased by at least two fold (SLR>1) in 4 of the 7 biopsies of Stage I tumours of the pancreas, in the Stage III biopsy and in the 2 stage IV biopsies (Table 5).

Differential analysis of the expression of the ednra gene in tumoral stages compared with the non-tumorous control (PA36), showed that expression levels of the ednra gene were higher in 3 of the 10 biopsies of pancreatic tumours (Table 6). Analysis of the differential expression of the ednra gene compared to the control with chronic pancreatitis (PA46) revealed that the expression levels of the ednra gene were increased by 3 (SLR=1.7) to 12 fold (SLR=3.7) in all the tumour biopsies (Table 7).

### 1.3. Discussion

These results showed that the expression levels of the efnb2 gene were increased in most of the stage I, and in all of stage III and IV, ductal adenocarcinomas of the pancreas, when compared with the non-tumorous control biopsy or with the chronic pancreatitis control biopsy, showing some degree of correlation between the stage of the disease and the level of gene expression.

These results also showed that the expression levels of the ednra gene were increased in all ductal adenocarcinomas of the pancreas, when compared with the non-tumorous control biopsy with pancreatitis, and the expression levels of the ednra gene were increased in 30% of ductal adenocarcinomas of the pancreas, when compared with the non-tumorous control biopsy without pancreatitis.

**Example 2.- Differential analysis of expression of the efnb2 gene and the ednra gene in samples of pancreas tissue using Real time - quantitative RT-PCR**

**2.1. Material and methods**

The method used consists in the reverse transcription of mRNA to cDNA followed by its amplification in a *LightCycler (Roche)* equipment using SYBR Green to detect the amplified product. Quantification was done in real time and permits the relative expression of the sequence to be calculated in different samples in the linear amplification phase of the reaction.

**Samples.** The samples studied were from clinically staged biopsies, obtained by surgical resection: biopsies of non-tumorous pancreatic tissue from individuals with ductal adenocarcinoma of the pancreas ("non-tumorous") (n = 1); biopsies of non-tumorous pancreatic tissue, but with pancreatitis, from individuals without ductal adenocarcinoma of the pancreas, but with chronic pancreatitis (n = 1) ("chronic pancreatitis"); biopsies of patients with ductal adenocarcinoma of the pancreas (n = 5) in one of the following stages: Stage I, tumour limited to the pancreas and Stage IV, metastases in distant tissues or organs. All the samples were classified histologically (grade and stage) in the Hospital Central de Asturias, the same hospital in which the samples had been collected according to the precepts of the Helsinki declaration. Samples were frozen in liquid nitrogen immediately after extraction and kept at -80°C until analysis.

Real Time Quantitative RT-PCR. The analysis was carried out with total RNA from individual subjects. The 7 samples analysed are described in Table 8.

Table 8.

| Description of the samples analysed. | | | |
|---|---|---|---|
| Code of sample | Type | TNM Classification | Stage |
| PA36 | Non-tumorous | - | - |
| PA46 | Chronic Pancreatitis | - | - |
| PA19 | Tumour | T1bN0M0 | I |
| PA03 | Tumour | T2N0M0 | I |
| PA33 | Tumour | T1bN0M0 | I |
| PA14 | Tumour | T3N1M1 | IV |
| PA23 | Tumour | T1bN1M1 | IV |

*CDNA synthesis*

The total RNA of each of the biopsies was obtained by homogenising the tissue in TRIzol® Reagent (Life Technologies), following the supplier's recommendations. The resulting total RNA was cleaned with the RNeasy kit (QIAGEN) (Chomczynski P. et at., Anal. Biochem., 1987, 162: 156; Chomczynski P., Biotechniques, 1993, 15: 532). The RNA was quantified spectrophotometrically and 5 μg of total RNA were digested with DNaseI. A total of 1 μg of RNA treated with DNAse was used as starter material for the synthesis of the first strand of cDNA with the inverse transcriptase enzyme SuperScript™ II RNase (Life Technologies), using an oligo-dT oligonucleotide that contained the sequence of the RNA polymerase promoter of the phage T7 as a primer. Aliquots of cDNA diluted to the working concentration were prepared.

*Amplification*

The cDNA synthesised was amplified using specific primers of the human efnb2 gene (5'-AGCTTGTTTAACGGCAGTGTCAT -3' and 5'-GCAGCAATTTGGCAACCTTT-3'), of the human ednra gene (5'-TGCTTGAATTGCAAGGCTAAGAA-3' and 5'-GCGCCAGTGGAATAATAGATTTG-3') and of the gene that codes for the human ribosomal protein L10 (5'-TGCGATGGCTGCACACA-3' and 5'-TCCCTTAGAGCAACCCATACAAC-3'). PCR reactions in real time were prepared using the *LightCycler-FastStart DNA master SYBR Green I kit (Roche)* following the manufacturer's instructions. The amplification programme consisted in 1 cycle of 95°C (denaturation) for 10 s, 60°C (annealing) for 5 s, 72°C (amplification and acquisition of fluorescence) for 10 s. The programme of analysis of the denaturation curves consists of a cycle of one pulse of 95°C, 65°C for 15 s, and a pulse of 95°C for the amplification and acquisition step.

*Quantification*

Firstly, the specificity of the PCR products are determined by analysing the denaturation curves. Subsequently, as a relative measure of gene expression, the ratio of the abundance of mRNA transcripts of efnb2 to the abundance of ribl10 transcripts was calculated and the ratio was normalised for each of the tumoral samples on the basis of the values of the control sample. To calculate the efficiency of the PCR reactions (efnb2 and ribl10) a reference curve was developed for each gene sequence with serial dilutions of cDNA. The concentrations of template cDNA for reactions in the reference curve were given the arbitrary values 10, 5, 2.5, 1.25 and 0.625. The efficiency was calculated using the following equation:

$$E = 10^{-1/p}$$

where $E$ is the efficiency of the amplification and $p$ is the gradient of the reference line.

The ratio of the values of gene expression was determined using the following equation, which relates the experimental data of the amplification and corrects the error resulting from the difference in efficiency of the PCR reactions:

$$\text{Ratio} = \frac{E_{target}^{-(Cp\ target\ control\ -Cp\ target\ sample\ )}}{E_{reference}^{-(Cp\ reference\ control\ -\ Cp\ reference\ sample)}}$$

where $E$ is the amplification efficiency, $Cp$ is the *Crossing point*, *target* is efnb2, *reference* is ribl10, *control* is the control sample (non-tumorous or chronic pancreatitis) and *sample* is the tumoral sample.
The same protocol was used to quantify ednra, in comparison with ribl10.

## 2.2. Results

### Analysis of the denaturation curves

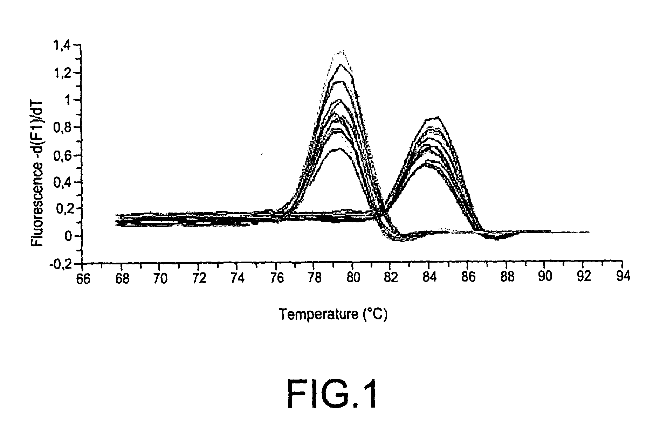

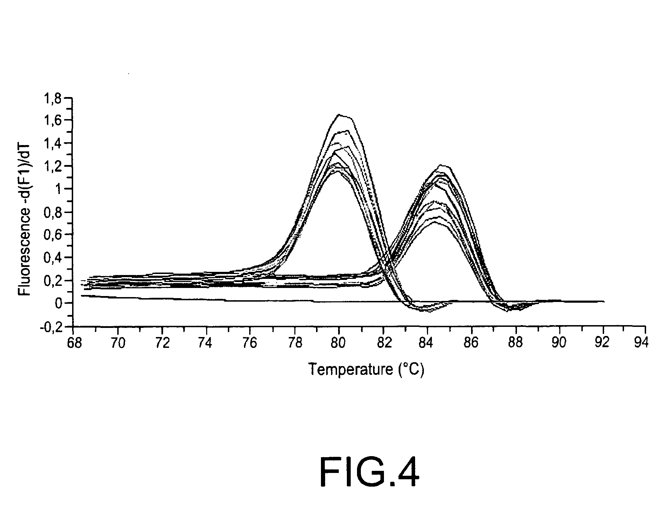

Analysis of the PCR products showed specific amplification of two products with denaturation temperatures similar to those expected according to the software used to design the primers, *PrimerExpress (Applied Biosystems)* (Figure 1 for efnb2; figure 4 for ednra).

### Calculation of the efficiency of the PCR reactions

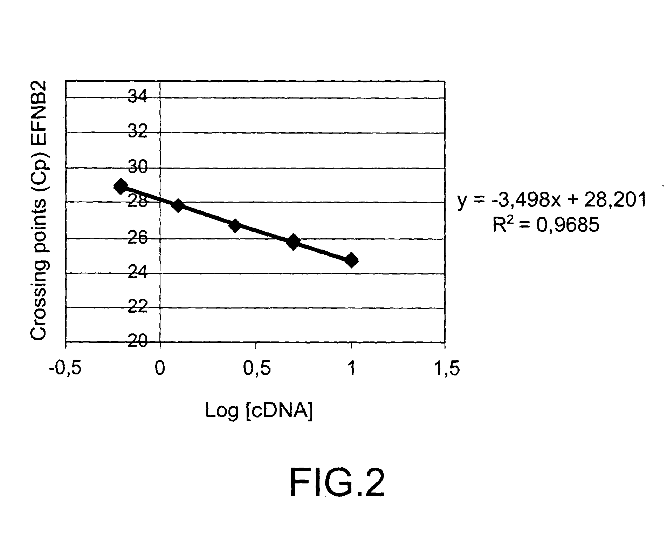

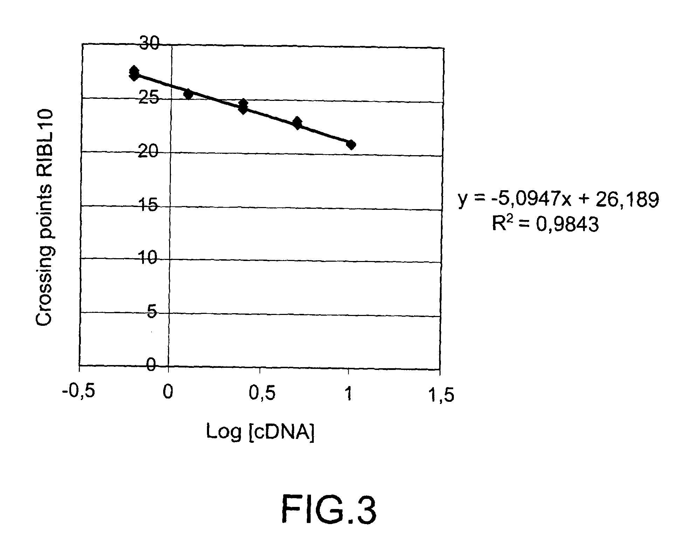

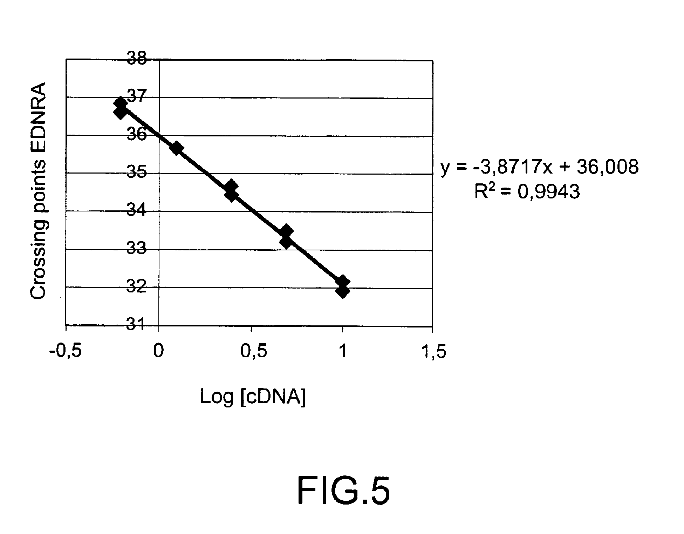

For each of the 5 dilutions of cDNA, two replicate samples were amplified and the cut off points (Cp) plotted on a graph against the logarithm of the concentration of cDNA to construct the reference line (Tables 9 and 10, figures 2 and 3 for efnb2; tables 13 and 14, figures 5 and 3 for ednra).

Table 9.

| Cut off points (Cp) of the amplification of 5 dilutions of cDNA for each replica amplified with primers of the efnb2 gene | | |
|---|---|---|
| [RNA] (ng) | Log [RNA] | Cp |
| 10 | 1 | 24.66 |
| 10 | 1 | 24.75 |
| 5 | 0.698970004 | 25.69 |
| 5 | 0.698970004 | 25.85 |
| 2.5 | 0.397940009 | 26.77 |
| 2.5 | 0.397940009 | 26.79 |
| 1.25 | 0.096910013 | 27.89 |
| 1.25 | 0.096910013 | 27.85 |
| 0.625 | -0.204119983 | 29 |

Table 9.   (continued)

| Cut off points (Cp) of the amplification of 5 dilutions of cDNA for each replica amplified with primers of the efnb2 gene | | |
|---|---|---|
| **[RNA] (ng)** | **Log [RNA]** | **Cp** |
| 0.625 | -0.204119983 | 28.84 |

Table 10.

| Cut off points (Cp) for the amplifications of 5 dilutions of cDNA for each replica amplified with primers of the ribl10 gene | | |
|---|---|---|
| **[RNA] (ng)** | **Log [RNA]** | **Cp** |
| 10 | 1 | 20.87 |
| 10 | 1 | 20.98 |
| 5 | 0.698970004 | 22.65 |
| 5 | 0.698970004 | 22.98 |
| 2.5 | 0.397940009 | 24.33 |
| 2.5 | 0.397940009 | 24.74 |
| 1.25 | 0.096910013 | 25.29 |
| 1.25 | 0.096910013 | 25.44 |
| 0.625 | -0.204119983 | 27.02 |
| 0.625 | -0.204119983 | 27.38 |

**Quantification of the change in expression of the efnb2 gene**

Two replicas of each sample (controls and tumours) were amplified with the specific primers of efnb2 and ribl10. From the cut off points generated in these amplifications, changes in expression of the efnb2 gene were calculated in tumorous samples compared to non-tumorous and chronic pancreatitis controls, applying the previously described equation (Tables 11 and 12).
The same protocol was followed to quantify ednra, in comparison with ribl10 (Tables 15 and 16).

Table 11.

| Experimental cut off points for ribl10 and efnb2 in 5 tumours and control samples. | | | | | | | |
|---|---|---|---|---|---|---|---|
| **ribl10** | | | | | | | |
| **Replicas** | **PA36** | **PA46** | **PA03** | **PA14** | **PA19** | **PA23** | **PA33** |
| 1 | 30.53 | 28.72 | 29.75 | 27.14 | 31.51 | 29.44 | 29.06 |
| **2** | 29.50 | 28.87 | 29.81 | 27.12 | 31.65 | 29.33 | 29.07 |
| *Mean* | *30.02* | *28.80* | *29.78* | *27.13* | *31.58* | *29.39* | *29.07* |
| *SD* | *0.73* | *0.11 1* | *0.04* | *0.01* | *0.10* | *0.08* | *0.01* |
| **efnb2** | | | | | | | |
| **Replicas** | **PA36** | **PA46** | **PA03** | **PA14** | **PA19** | **PA23** | **PA33** |
| 1 | 31.13 | 28.84 | 27.73 | 24.89 | 28.43 | 27.28 | 28.23 |
| **2** | 31.82 | 29.06 | 27.80 | 24.89 | 28.60 | 26.53 | 28.05 |
| *Mean* | *31.48* | *28.95* | *27.77* | *24.89* | *28.52* | *26.91* | *28.14* |
| *SD* | *0.49* | *0.16* | *0.05* | *0.00* | *0.12* | *0.53* | *0.13* |

Table 12.

| Values of overexpression (Fold Change) of efnb2 in 5 tumors, compared with PA36 non-tumorous control sample and compared with PA46 chronic pancreatitis control sample. | | | | | |
|---|---|---|---|---|---|
| Samples | PA03 | PA14 | PA19 | PA23 | PA33 |
| Increased expression relative to PA36 non-tumorous control sample | 10.3 | 20.7 | 14.2 | 15.2 | 5.8 |
| Increased expression relative to PA46 chronic pancreatitis control sample | 5.3 | 13.0 | 7.0 | 8.6 | 4.6 |

Table 13.

| Cut off points (Cp) of the amplification of 5 dilutions of cDNA for each replica amplified with the primers of the ednra gene | | |
|---|---|---|
| [RNA] (ng) | Log [RNA] | Cp |
| 10 | 1 | 32.16 |
| 10 | 1 | 31.95 |
| 5 | 0.698970004 | 33.48 |
| 5 | 0.698970004 | 33.21 |
| 2.5 | 0.397940009 | 34.44 |
| 2.5 | 0.397940009 | 34.67 |
| 1.25 | 0.096910013 | 35.66 |
| 1.25 | 0.096910013 | 35.64 |
| 0.625 | -0.204119983 | 36.85 |
| 0.625 | -0.204119983 | 36.61 |

Table 14.

| Cut off points (Cp) of the amplification of the 5 dilutions of cDNA for each replica amplified with the primers of the ribl10 gene | | |
|---|---|---|
| [RNA] (ng) | Log [RNA] | Cp |
| 10 | 1 | 20.87 |
| 10 | 1 | 20.98 |
| 5 | 0.698970004 | 22.65 |
| 5 | 0.698970004 | 22.98 |
| 2.5 | 0.397940009 | 24.33 |
| 2.5 | 0.397940009 | 24.74 |
| 1.25 | 0.096910013 | 25.29 |
| 1.25 | 0.096910013 | 25.44 |
| 0.625 | -0.204119983 | 27.02 |
| 0.625 | -0.204119983 | 27.38 |

Table 15.

| Experimental cut off points for ribl10 and ednra in 5 tumours and control samples. | | | | | | | |
|---|---|---|---|---|---|---|---|
| **rib110** | | | | | | | |
| **Replicas** | **PA36** | **PA46** | **PA03** | **PA14** | **PA19** | **PA23** | **PA33** |
| 1 | 26.94 | 27.37 | 27.81 | 24.93 | 29.99 | 27.94 | 27.15 |
| *2* | *27.72* | *27.46* | *28.33* | *25.04* | *29.38* | *27.23* | *27.66* |
| *Mean* | *27.33* | *27.42* | *28.07* | *24.99* | *29.69* | *27.59* | *27.41* |
| *SD* | *0.55* | *0.06* | *0.37* | *0.08* | *0.43* | *0.50* | *0.36* |
| **endra** | | | | | | | |
| **Replicas** | **PA36** | **PA46** | **PA03** | **PA14** | **PA19** | **PA23** | **PA33** |
| 1 | 30.67 | 30.73 | 27.60 | 26.59 | 29.85 | 27.35 | 28.66 |
| 2 | 29.50 | 30.73 | 27.86 | 26.46 | 28.80 | 27.42 | 29.09 |
| *Mean* | *30.09* | *30.73* | *27.73* | *26.53* | *29.33* | *27.39* | *28.88* |
| SD | *0.83* | *0.00* | *0.18* | *0.09* | *0.74* | *0.05* | *0.30* |

Table 16.

| Overexpression values (Fold Change) of ednra in 5 tumors, compared with PA36 non-tumorous control sample and compared with PA46 chronic pancreatitis control sample. | | | | | |
|---|---|---|---|---|---|
| **Samples** | **PA03** | **PA14** | **PA19** | **PA23** | **PA33** |
| Increased expression relative to PA36 non-tumorous control sample) | 5.7 | 2.9 | 4.6 | 5.6 | 2.1 |
| Increased expression relative to PA46 pancreatitis control sample | 4.6 | 14.9 | 3.5 | 1.7 | 2.1 |

The results confirmed the data obtained by measuring the difference in gene expression with DNA chips (example 1), that is, the expression of the efnb2 and ednra genes was significantly increased in the tumours, compared to non-tumorous samples and compared to pancreatitis samples; the average increase of efnb2 gene expression was 13 times, when compared to non-tumorous samples without pancreatitis, and 8 times, when compared with non-tumorous samples with chronic pancreatitis; the average increase of ednra gene expression was 4 times, when compared to non-tumorous samples without pancreatitis, and 5 times, when compared with non-tumorous samples with chronic pancreatitis

**2.3. Discussion**

These results confirmed that the increased expression of efnb2 and ednra is associated to the ductal adenocarcinoma of the pancreas. In the particular case of ednra, the levels of gene expression measured by quantitative RT-PCR were more homogeneous between tumours of different stages than when measured with DNA chips.

**Example 3.- Differential analysis of expression of the EFNB2 protein in samples of pancreas tissue using the Western blot technique with specific antibodies.**

**3.1 Material and methods.**

**Samples:** The samples studied were from clinically staged biopsies, obtained by surgical resection: biopsies of non-tumorous pancreatic tissue from individuals without ductal adenocarcinoma of the pancreas (n = 2, samples number 15 and 42); biopsies of non-tumorous pancreatic tissue from individuals with ductal adenocarcinoma of the pancreas (n = 4, samples number 34, 35, 36 and 37); biopsies of non-tumorous, but inflamed, pancreatic tissue, from individuals with ductal adenocarcinoma of the pancreas and chronic pancreatitis (n = 3, samples number 28, 29 and 30); biopsies of tumorous pancreatic tissue, from individuals with ductal adenocarcinoma of the pancreas stage I (n = 2, samples number 27 and 33) and stage IV (n = 1, sample number 23); one biopsy of tumorous lung tissue, from an individual with metastasic ductal adenocarcinoma of the pancreas (n = 2, sample number 38) and one biopsy of non-tumorous lung tissue, from the same individual; cell extracts from immortalised ductal cell cultures. All samples were classified histologically (grade and stage) in the Hospital Central de Asturias, the same hospital from which the samples had been collected, following the precepts of the Helsinki declaration. The samples were frozen in liquid nitrogen immediately after extraction and stored at -80°C until analysis.

**Protein extraction:** Frozen samples were homogenised in RIPA B buffer [sodium phosphate 20 mM (pH 7.4), NaCl 150 mM, Triton X-100 1%, EDTA 5 mM and a combination of protease inhibitors (Roche Diagnostics Inc., Mannheim,Germany)]. Samples were then centrifuged at 15000 x g for 10 minutes at 4°C, to eliminate cellular debris. Then, the supernatant was recovered and the concentration of proteins was estimated by the Bradford assay (Bio-Rad, Hercules, CA, US).

**Western transfer assays:** Samples of protein extracts with 40 µg of total protein were taken, SDS-PAGE stacking buffer was supplemented with 5% of β-mercaptoethanol and were incubated at 100°C for 5 min after which they were packed in an 8% polyacrylamide gel. The proteins were then transferred to nitrocellulose membranes and probed with specific antibodies against the EFNB2 protein (Santacruz, USA). Finally, the membranes were hybridised with a secondary antibody conjugated with peroxidase (Amersham, Little Chalfont, UK) and the chemoluminescent signal was detected using the ECL system (Amersham) with high performance chemiluminescence film (Hyperfilm ECL, Amersham).

### 3.2 Results

**Expression of EFNB2 in human ductal adenocarcinomas of the pancreas:** The results obtained are compiled in Fig. 6 and in Table 17. As can be observed, expression levels of the EFNB2 protein were almost undetectable, or were undetectable, in biopsies of non-tumorous pancreatic tissue from individuals without ductal adenocarcinoma of the pancreas, biopsies of non-tumorous pancreatic tissue from individuals with ductal adenocarcinoma of the pancreas, or biopsies of non-tumorous, but inflamed, pancreatic tissue, from individuals with ductal adenocarcinoma of the pancreas and chronic pancreatitis. In contrast, EFNB2 was overexpressed in 2 of the 3 ductal adenocarcinomas of the pancreas that were analyzed. The major immunoreactive band was situated at 120 kDa, which corresponded to the mature form of EFNB2 (Tabla 17, Figure 6).

### 3.3. Discussion

The results confirmed, at the protein expression level, the data obtained by measuring the difference in gene expression, i.e. expression of the EFNB2 protein was greatly increased in tumour samples compared to non-tumorous samples from individuals with or without pancreatitis.

Table 17.

| Expression of EFNB2 in human pancreatic adenocarcinomas | | | | |
|---|---|---|---|---|
| **Type of sample** | *n* | **EFNB2** absent | **EFNB2** present | **EFNB2** overexpressed |
| Pancreas samples of healthy individuals (PA15, PA42) | 2 | 2 | 0 | 0 |
| Non-tumorous pancreas samples of individuals with tumour (PA34, PA35, PA36, PA37) | 4 | 4 | 0 | 0 |
| Non-tumorous inflamed pancreas samples of individuals with pancreatitis and tumour (PA28, PA29, PA30) | 3 | 3 | 0 | 0 |
| Pancreatic ductal adenocarcinoma stage IV. b (PA 23) | 1 | 0 | 1 | 1 |
| Pancreatic ductal adenocarcinoma Stage I (PA27, PA33) | 2 | 1 | 1 | 1 |

Table 17.   (continued)

| Expression of EFNB2 in human pancreatic adenocarcinomas | | | | |
|---|---|---|---|---|
| **Type of sample** | *n* | **EFNB2** absent | **EFNB2** present | **EFNB2** overexpressed |
| Pulmonary metastasis of a ductal adenocarcinoma of the pancreas (PA39) | 1 | 1 | 0 | 0 |
| Non-tumorous lung sample of individuals with pulmonary metastasis (PA38) | 1 | 1 | 0 | 0 |
| Immortalised pancreatic ductal cell lines (Capan-1, BxPC-3, PANC-1) | 3 | 3 | 0 | 0 |

## Claims

1. An *In vitro* method to detect the presence of a cancer of the pancreas in an individual, to determine the stage or severity of this cancer in an individual or to monitor the effect of the therapy administered to the individual with this cancer, that comprises:

   a) detection and/or quantification either of the EFNB2 protein, of the mRNA of the efnb2 gene, or of the corresponding cDNA, or of the EDNRA protein, of the mRNA of the ednra gene, or of the corresponding cDNA, or of any combination of these in a sample of the said individual, and
   b) comparison of either of the amount of EFNB2 protein, of the mRNA of the efnb2 gene or of the corresponding cDNA, or of the amount of the EDNRA protein, of the mRNA of the ednra gene, or of the corresponding cDNA or of any combination of these detected in a sample of an individual, with the respective amount either of the EFNB2 protein, of the mRNA of the efnb2 gene, or of the corresponding cDNA, or with the amount of the EDNRA protein, of the mRNA of the ednra gene or of the corresponding cDNA, or with any combination of these, detected in the samples of control individuals or in previous samples of the same individual or with normal reference values.

2. Method according to claim 1 in which the cancer of the pancreas is a ductal adenocarcinoma of the pancreas.

3. Method according to claim 1 in which this sample is a sample of pancreas tissue.

4. Method according to claim 3 in which this sample of pancreas tissue to be analysed is obtained by any conventional method, preferably by surgical resection.

5. Method according to claim 1 in which this sample is a sample of urine, blood, plasma, serum, gastric juice aspirate, bile or semen.

6.  Method according to claim 1, in which the sample to be analysed is taken from an individual not previously diagnosed with cancer.

7.  Method according to claim 1 in which the sample to be analysed is obtained from an individual who has been previously diagnosed with a cancer, preferably in the pancreas.

8.  Method according to claim 1 in which the sample to be analysed is obtained from an individual receiving treatment, or who has been treated previously against cancer, preferably of the pancreas.

9.  Method according to claim 1 **characterised in that** it comprises the extraction of the sample, either to obtain an extract of proteins or an extract of total RNA.

10. Method according to claim 9 **characterised in that** the detection of the EFNB2 protein, or of the EDNRA protein comprises a first step in which the protein extract of the sample is placed in contact with a composition of one or more specific antibodies against one or more epitopes of the EFNB2 protein or of the EDNRA protein, and a second step in which the complexes formed by the antibodies and the EFNB2 protein or the EDNRA protein are quantified.

11. Method according to claim 10, **characterised in that** these antibodies correspond to monoclonal or polyclonal antibodies, intact or recombinant fragments of antibodies, combibodies and Fab or scFv antibody fragments, specific against the EFNB2 protein or against the EDNRA protein; these antibodies are human, humanised or of non-human origin.

12. Method according to claims 10 or 11 **characterised in that** in the quantification of the complexes formed by antibodies and the EFNB2 protein or the EDNRA protein, the techniques used are selected from the group comprised by: western-blot, ELISA (Enzyme-Linked Immunosorbent assay), RIA (Radioimmunoassay), Competitive EIA (Competitive Enzyme Immunoassay), DAS-ELISA (Double Antibody Sandwich-Elisa), immunocytochemical or immunohistochemical techniques, techniques based on the use of biochips or protein microarrays that include specific antibodies, assays based on the precipitation of colloidal gold in formats such as *dipsticks;* or by affinity chromatography techniques, ligand binding assays or lectin binding assays.

13. Method according to claim 9 **characterised in that** the detection either of the mRNA or of the corresponding cDNA of the efnb2 gene, or of the mRNA or of the corresponding cDNA of the ednra gene, comprises a first step of amplification of the mRNA that is present in the extract of total RNA, or of the corresponding cDNA synthesised by reverse transcription of the mRNA; and a second quantification step of the amplification product from either the mRNA or the cDNA of the efnb2 gene, or the mRNA or the cDNA of the ednra gene.

14. Method according to claim 13 **characterised in that** the amplification is performed qualitatively or quantitatively, by RT-PCR using primer oligonucleotides where the sequences of the primers used to amplify the sequence of the efnb2 gene are 5'AGCTTGTTTAACGGCAGTGTCAT-3' and 5'-GCAGCAATTTGGCAACCTTT-3' and the sequences of primers used to amplify the sequence of the ednra gene are 5'-TGCTTGAATTGCAAGGCTAAGAA-3' and 5'-GCGCCAGTGGAATAATAGATTTG -3'.

15. Method according to claim 9 **characterised in that** the detection is done with specific probes either of mRNA or of the corresponding cDNA of the ednra gene, by techniques such as northern-blot or northern transfer.

16. Method according to claim 9 **characterised in that** the detection of the mRNA is done by Real time quantitative RT-PCR (Q-PCR).

17. Use of nucleotide or peptide sequences derived from the efnb2 gene or the ednra gene to detect *in vitro* the presence of a cancer of the pancreas, especially of a ductal adenocarcinoma of the pancreas, to determine *in vitro* the stage or severity of this cancer in the individual, or to monitor *in vitro* the effect of the therapy administered to an individual with this cancer.

18. *In vitro* method to identify and evaluate the efficacy of therapeutic compounds against a cancer of the pancreas, especially against a ductal adenocarcinoma of the pancreas that comprises:

a) placing in contact a culture of endothelial cells with the candidate compound in the appropriate conditions and for a suitable time for these to interact,

b) detecting and quantifying expression levels of the EFNB2 gene, the protein EFNB2, the ednra gene, the EDNRA protein or any combination of these, and

c) comparing the effects of these expression levels with those of control cultures of endothelial cells not treated with the candidate compound.

19. Use of the nucleotide or peptide sequence derived from the efnb2 gene and/or the ednra gene, in methods to screen for, identify, develop and evaluate the efficiency of compounds to treat a cancer of the pancreas, especially a ductal adenocarcinoma of the pancreas.

20. An agent **characterised in that** it inhibits the expression and/or activity of the EFNB2 protein and/or the EDNRA protein or **in that** it inhibits the carcinogenic effects of induction of expression of the EFNB2 protein and/or the EDNRA protein.

21. An agent according to claim 20 selected from the group comprised by:

a) an antibody, or combination of antibodies, specific against one or more epitopes present in the EFNB2 protein or in the EDNRA protein, preferably a human or humanised monoclonal antibody; can also be a fragment of an antibody, a single chain antibody or an anti-idiotype antibody,

b) cytotoxic agents such as toxins, molecules with radioactive atoms or chemotherapeutic agents, including but not limited to small organic and inorganic molecules, peptides, phosphopeptides, antisense molecules, ribozymes, triple helix molecules, RNA interference (*small interfering RNA*), double stranded RNA etc., which inhibit expression and/or activity of the EFNB2 protein and/or the EDNRA protein and

c) antagonistic compounds of the EFNB2 protein and/or the EDNRA protein, which inhibit one or more of the functions of the EFNB2 protein and/or the EDNRA protein.

22. Agent according to claims 20 or 21 to treat a cancer of the pancreas, especially a ductal adenocarcinoma of the pancreas.

23. Use of any of the agents according to claims 20 or 21 to elaborate a medicinal product to treat a cancer of the pancreas, especially a ductal adenocarcinoma of the pancreas.

24. Pharmaceutical composition that consists of a therapeutically effective amount of one or several agents according to claims 20 or 21 in combination with at least one pharmaceutically acceptable agent.

25. Pharmaceutical composition according to claim 24 characterised because it contains another active ingredient, preferably one that inhibits the function of the EFNB2 protein and/or the EDNRA protein.

FIG.1

FIG.2

$$y = -5{,}0947x + 26{,}189$$
$$R^2 = 0{,}9843$$

FIG.3

FIG.4

$y = -3,8717x + 36,008$
$R^2 = 0,9943$

FIG.5

FIG.6

## European Patent Office

## PARTIAL EUROPEAN SEARCH REPORT

**Application Number**

which under Rule 45 of the European Patent Convention EP 03 38 0236
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | LIU W ET AL: "COEXPRESSION OF EPHRIN-BS AND THEIR RECEPTORS IN COLON CARCINOMA" CANCER, AMERICAN CANCER SOCIETY, PHILADELPHIA, PA, US, vol. 94, no. 4, 15 February 2002 (2002-02-15), pages 934-939, XP001156840 ISSN: 0008-543X | 20-22, 24,25 | G01N33/574 |
| A | * the whole document * | 1-19,23 | |
| A | KATAOKA, H. ET AL: "Expression profile of EFNB1, EFNB2, two ligands of EPHB2 in human gastric cancer" J CANCER RES CLIN ONCOL, vol. 128, 2002, pages 343-348, XP001179752 * abstract * | 1-25 | |
| X | WO 02/26827 A (NOVARTIS) 4 April 2002 (2002-04-04) | 20-22, 24,25 | |
| A | * the whole document * | 1-19,23 | |

-/--

**TECHNICAL FIELDS SEARCHED (Int.Cl.7)**

G01N

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 20 September 2004 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent
Office

**PARTIAL EUROPEAN SEARCH REPORT**

Application Number

EP 03 38 0236

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | US 2002/119097 A1 (GALE, N.W AND YANCOPOULUS, G.D.) 29 August 2002 (2002-08-29) | 20-22, 24,25 | |
| A | * the whole document * | 1-19,23 | |
| A,D | IACOBUZIO-DONAHUE, C.A. ET AL: "Exploration of global gene expression patterns in pancreatic adenocarcinoma using cDNA microarrays" AMERICAN JOURNAL OF PATHOLOGY, vol. 162, no. 4, April 2003 (2003-04), pages 1151-1162, XP009027111 * the whole document * | 1-25 | |
| X | HOTZ, H.G. ET AL: "The effect of selective endothelin-A-receptor blockade on human pancreatic cancer in vivo" PANCREAS, vol. 21, no. 4, 2000, page 448, XP009036713 * the whole document * | 1-25 | **TECHNICAL FIELDS SEARCHED** (Int.Cl.7) |
| X | MAITRE NATHALIE L ET AL: "Endothelin receptor A (ETA) expressing tumors as potential targets for endothelin receptor antagonist therapy" PROCEEDINGS OF THE AMERICAN ASSOCIATION FOR CANCER RESEARCH ANNUAL MEETING, no. 41, March 2000 (2000-03), page 528, XP001183532 & 91ST ANNUAL MEETING OF THE AMERICAN ASSOCIATION FOR CANCER RESEARCH.; SAN FRANCISCO, CALIFORNIA, USA; APRIL 01-05, 2000 ISSN: 0197-016X * the whole document * | 1-25 | |

-/--

EPO FORM 1503 03.82 (P04C10)

**European Patent Office**

## PARTIAL EUROPEAN SEARCH REPORT

Application Number

EP 03 38 0236

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | WO 00/34788 A (BOARD OF REAGENTS, THE UNIVERSITY OF TEXAS SYSTEM) 15 June 2000 (2000-06-15) | 20-22, 24,25 | |
| A | * the whole document * | 1-19,23 | |
| X | US 6 545 048 B1 (CALIFORNIA INSTITUTE OF TECHNOLOGY) 8 April 2003 (2003-04-08) * claims 1-16 * | 20,24,25 | |

TECHNICAL FIELDS SEARCHED (Int.Cl.7)

EPO FORM 1503 03.82 (P04C10)

**European Patent**
**Office**

INCOMPLETE SEARCH
SHEET C

Application Number

EP 03 38 0236

Claim(s) searched incompletely:
    1-25

Reason for the limitation of the search:

Present claims 20-25 relate to an extremely large number of possible compounds defined by their property of inhibiting the expresion or activity of the EFNB2 or the EDNRA protein. Support within the meaning of Article 84 EPC and disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely the antibodies against the EFNB2 protein and antibodies and non-defined "inhibitors" of the EDNRA protein.

European Patent
Office

Application Number

EP 03 38 0236

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**European Patent Office**

**LACK OF UNITY OF INVENTION
SHEET B**

Application Number

EP 03 38 0236

The Search Division considers that the present European patent application does not comply with the requirements of unity of invention and relates to several inventions or groups of inventions, namely:

1. claims: 1-25 (partially)

    Methods for detecting, staging and monitoring the therapy of cancer of pancreas, comprising detecting the expression of the EFNB2 (Ephrin B2) gene and agents and compositions for the treatment of cancer of pancreas containing an inhibitor of the expression of the EFNB2 (Ephrin B2) gene.

    ---

2. claims: 1-25 (partially)

    Methods for detecting, staging and monitoring the therapy of cancer of pancreas, comprising detecting the expression of the EDNRA (endothelin receptor A) gene and agents and compositions for the treatment of cancer of pancreas containing an inhibitor of the expression of the EDNRA gene.

    ---

## ANNEX TO THE EUROPEAN SEARCH REPORT
## ON EUROPEAN PATENT APPLICATION NO.

EP 03 38 0236

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2004

| Patent document cited in search report | | Publication date | Patent family member(s) | | Publication date |
|---|---|---|---|---|---|
| WO 0226827 | A | 04-04-2002 | AU<br>WO | 1229202 A<br>0226827 A1 | 08-04-2002<br>04-04-2002 |
| US 2002119097 | A1 | 29-08-2002 | WO | 02058538 A2 | 01-08-2002 |
| WO 0034788 | A | 15-06-2000 | AU<br>CA<br>EP<br>WO<br>US | 3105700 A<br>2354084 A1<br>1137945 A1<br>0034788 A1<br>2002164663 A1 | 26-06-2000<br>15-06-2000<br>04-10-2001<br>15-06-2000<br>07-11-2002 |
| US 6545048 | B1 | 08-04-2003 | AU<br>CA<br>EP<br>WO | 6065000 A<br>2376912 A1<br>1280552 A2<br>0100198 A2 | 31-01-2001<br>04-01-2001<br>05-02-2003<br>04-01-2001 |

EPO FORM P0459

For more details about this annex : see Official Journal of the European Patent Office, No. 12/82